# EUROPEAN PATENT APPLICATION

(11) **EP 4 074 408 A1**
(43) Date of publication of application: **19.10.2022**
(21) Application number: 21168186.1
(22) Date of filing: 13.04.2021
(51) Int. Cl.: B01D 53/14, C07D 301/10, C07C 29/60

(54) **IMPROVED CYCLE GAS PROCESS FOR PREPARATION OF ETHYLENE OXIDE FROM ETHANOL**

(71) Applicant: Clariant International Ltd, 4132 Muttenz (CH)
(72) Inventor: FRANKE, Oliver, 81671 München (DE); OTROMKE, Malte, 68163 Mannheim (DE); BRAND, Stefan, 69493 Hirschberg-Leutershausesn (DE); WACHSEN, Olaf, 84518 Garching (DE)
(74) Representative: Kuba, Stefan

(57) **Abstract**

The invention relates to a cycle gas apparatus 1 for preparation of ethylene oxide from ethylene in a cycle gas process, comprising: a) an epoxidation reactor 2 containing a catalyst suitable for conversion of ethylene to ethylene oxide, having an inlet for a reactant gas and an outlet for the product gas 3, b) a first separator stage 7 suitable for removing ethylene oxide from the cycle gas process and providing it in a product stream 10, where the first separator stage 7 has an inlet connected to the outlet from the epoxidation reactor 2 and has an outlet by which the remaining gases can be returned to the cycle gas process, c) a second separator stage 12 suitable for removing CO₂ from the cycle gas process, where the second separator stage 12 has an inlet which is connected to the outlet of the first separator stage 7 and has an outlet by which the remaining gases can be returned to the cycle gas process, d) one or more gas inlets 15, 16 that allow introduction of O₂ and a ballast gas into the cycle gas apparatus 1, characterized in that there is a reactant stream inlet 4 between the outlet from the epoxidation reactor 2 and the first separator stage 7, which permits the introduction of ethylene into the cycle gas apparatus 1. The invention also relates to a process for preparing ethylene oxide from ethylene with the cycle gas apparatus 1 according to the invention, wherein an ethylene-containing reactant stream 5 is introduced into the cycle gas apparatus 1, and an ethylene oxide-containing product stream 10 is obtained.

## Description

The invention relates to a cycle gas apparatus 1 for preparation of ethylene oxide from ethylene in a cycle gas process, comprising:
a) an epoxidation reactor 2 containing a catalyst suitable for conversion of ethylene to ethylene oxide, having an inlet for a reactant gas and an outlet for the product gas 3,
b) a first separator stage 7 suitable for removing ethylene oxide from the cycle gas process and providing it in a product stream 10, where the first separator stage 7 has an inlet connected to the outlet from the epoxidation reactor 2 and has an outlet by which the remaining gases can be returned to the cycle gas process,
c) a second separator stage 12 suitable for removing CO₂ from the cycle gas process, where the second separator stage 12 has an inlet which is connected to the outlet of the first separator stage 7 and has an outlet by which the remaining gases can be returned to the cycle gas process,
d) one or more gas inlets 15, 16 that allow introduction of O₂ and a ballast gas into the cycle gas apparatus 1,
characterized in that there is a reactant stream inlet 4 between the outlet from the epoxidation reactor 2 and the first separator stage 7, which permits the introduction of ethylene into the cycle gas apparatus 1.

The invention further relates to a cycle gas process for preparing ethylene oxide from ethylene, in which a cycle gas apparatus 1 of the invention is operated, by feeding in ethylene between the outlet from the epoxidation reactor 2 and the first separator stage 7.

Ethanol can be obtained by the fermentation of biomass, for example of starch- or sugarcontaining plants. It is desirable here to use cellulose-containing waste materials as a sustainable and inedible source, called second-generation bioethanol (S. Kim, B. E. Dale, Biomass Bioenergy, 26 (2004) 361). The production costs of ethanol have decreased significantly in the last decade, particularly as a result of continuous industrial progress (R. Wooley, M. Ruth, D. Glassner, J. Sheehan, Biotechnol. Prog., 15 (1999) 794). As a result of the low-cost and high production capacities, bioethanol is added to gasoline in high proportions, reducing dependence on fossil sources. However, bioethanol is not just a renewable fuel but also a versatile chemical starting material: for instance, ethanol can enter into various types of reaction and can be converted, for example, to acetaldehyde (oxidation), ethyl acetate (oxidation and subsequent esterification), synthesis gas (gasification) or ethylene (dehydration). The latter can be reacted with oxygen to give ethylene oxide in an industrially very important conversion by an epoxidation (oxidation).

Ethylene oxide is the most important and also most valuable among the various molecules that can be obtained from ethanol. It is used for the production of ethylene glycol, polyethylene glycol and polyester fibres, and also plastic from polyethylene terephthalate (PET) (Z.L. Arsenijević, B.V. Grbić, N.D. Radić, B. Grbav̌cić, Chem. Eng. J. 116 (2006) 173-178). Nowadays, ethylene oxide is obtained almost exclusively from ethylene from fossil sources. The production of ethylene oxide is therefore not sustainable and highly dependent on the price of fossil sources (K. Weissermel, H-J. Arpe, Ind. Org. Chem. (4th ed.) (2003) 145-148). The idea of using ethylene oxide prepared from the renewable raw material ethanol has therefore gained much attention. There have been reports of the direct oxidation of ethanol to ethylene oxide with aluminium oxide-supported silver, copper and gold catalysts (M.J. Lippits, B.E. Nieuwenhuys, Catal. Today, 154 (2010) 127-132; M.J. Lippits, B.E. Nieuwen-huys, J. Catal., 274 (2010) 142-149). At present, however, these catalysts for direct oxidation do not attain the yield necessary for an industrial application. However, a promising prospect for industrial application is the 2-stage process consisting of the dehydration of ethanol and the subsequent oxidation of the ethylene thus obtained. In this 2-stage process, acidic catalysts are used in the first step for dehydration of the ethanol. The ethylene thus obtained is then converted to ethylene oxide in a second, separate step using the customary silver-containing oxidation catalysts as also used in the oxidation of ethylene from fossil sources.

Examples of silver catalysts that may be used in the preparation of ethylene oxide by the oxidation of ethylene at elevated temperatures in the presence of oxygen can be found, for example, in US 3878126A and US 4774222A.

WO 2014 018474 A1 discloses a process for preparing ethylene oxide, comprising: providing one or more charge components, where the one or more charge components contain at least ethylene that has been obtained by dehydration of ethanol; contacting the one or more charge components with a desulfurization catalyst comprising a support of high surface area and an amount of silver, where at least 20% of the silver is in the form of oxidized silver; and contacting the one or more charge components with a silver-containing epoxidation catalyst disposed in an ethylene oxide reactor, in order to form a reaction gas comprising ethylene oxide.

BR 8200311 A discloses a process wherein the conversion of ethanol to ethylene oxide is performed in two steps within a single reactor. The reaction step of dewatering the ethanol to ethylene is effected by contacting the ethanol that has been mixed with diluent gases and recirculation gases with the catalytic fluidized bed which is present within the reactor housing and outside the connected reaction tubes of the shell-and-tube system. There is a rigid bed of an oxidation catalyst within these reaction tubes. After being passed through the fluidized bed, the gases, now comprising ethylene and an oxygen stream or air, are guided into the upper part of the reactor and guided into the interior of the reaction tubes. Contact with the catalytic bed oxidizes the ethylene to ethylene oxide. The heat released in the oxidation is passed through the reaction tubes to the catalytic fluidized bed. The performance of the two reaction steps within a single reactor combines the advantageous effects of the two reaction steps. On the one hand, the heat that results from the oxidation reaction is used for performance of the endothermic ethanol dehydration reaction, which leads to a saving of fuel/oil or the saving of another energy source. On the other hand, the fluidized bed in which the dehydration reaction takes place is a very good heat absorber which is required for the control of the oxidation reaction, which leads to saving of thermal exchange surfaces and the saving of cooling fluids that are used in other processes.

On an industrial scale, the oxidation of ethylene to ethylene oxide is conducted in what are called cycle gas apparatuses; ethylene is oxidized here in an oxidation reactor in the presence of silver catalysts, and the resultant ethylene oxide is removed from the product stream together with by-products in a separator stage. After the removal of CO₂ in a further separator stage, the remaining stream is enriched again with ethylene, in order to be returned ("recycled") to the oxidation reactor. Such cycle gas processes are described, for example, in the publication Adam Chan, NexantThinking, Process Evaluation/Research Planning, "Ethylene Oxide and Derivatives", 2014-1, Section 3, p. 28-46.

It was an object of the invention to provide an improved cycle gas apparatus 1 and a corresponding process for preparing ethylene oxide from ethylene, wherein the process performed in the cycle gas apparatus is more tolerant with regard to impurities in the ethylene reactant used.

This object is achieved by a cycle gas apparatus 1 for preparation of ethylene oxide from ethylene in a cycle gas process, comprising:
a) an epoxidation reactor 2 containing a catalyst suitable for conversion of ethylene to ethylene oxide, having an inlet for a reactant gas and an outlet for the product gas 3,
b) a first separator stage 7 suitable for removing ethylene oxide from the cycle gas process and providing it in a product stream 10, where the first separator stage 7 has an inlet connected to the outlet from the epoxidation reactor 2 and has an outlet by which the remaining gases can be returned to the cycle gas process,
c) a second separator stage 12 suitable for removing CO₂ from the cycle gas process, where the second separator stage 12 has an inlet which is connected to the outlet of the first separator stage 7 and has an outlet by which the remaining gases can be returned to the cycle gas process,
d) one or more gas inlets 15, 16 that allow introduction of O₂ and a ballast gas into the cycle gas apparatus 1,
characterized in that there is a reactant stream inlet 4 between the outlet from the epoxidation reactor 2 and the first separator stage 7, which permits the introduction of ethylene into the cycle gas apparatus 1.

The cycle gas apparatus here is configured such that the outlet from the second separator stage 12 is connected to the inlet for the reactant gas 17 from the epoxidation reactor 2, in order to enable recycling of the cycle gas as reactant gas.

The invention also relates to a cycle gas process for preparing ethylene oxide from ethylene, in which a cycle gas apparatus 1 of the invention is operated, by feeding in ethylene between the outlet from the epoxidation reactor 2 and the first separator stage 7.

The epoxidation reactor 2 may be of any reactor type capable of converting ethylene to ethylene oxide, wherein the ethylene is guided into the reactor via the reactor inlet together with the reactant gas 17, and the ethylene oxide formed is discharged from the reactor via the outlet together with the product gas 3. The epoxidation reactor 2 is preferably a fixed bed reactor in tubular form, through which the reaction gases flow in the direction of the longitudinal axis, which is temperature-controllable and which contains a catalyst that catalyzes the epoxidation of ethylene. The catalyst is typically a silver catalyst, wherein the silver is applied in dispersed form to an Al₂O₃ support and wherein the catalyst is in the form of catalyst particles in the fixed bed of the reactor. The reactor is temperature-controllable, such that the catalyst in the reactor can be heated together with the reaction gases to a temperature between 140°C and 300°C, preferably between 210°C and 280°C, more preferably between 220°C and 260°C, and can be kept at that temperature. The cycle gas can be preheated upstream of the reactor by means of a heat exchanger, for example to a temperature between 120°C and 160°C.

The cycle gas apparatus is configured such that, during the cycle gas process, the reactant gas 17 includes generally between 21 mol% and 51 mol% of ethylene and 3 mol% to 13 mol% of O₂ and more preferably between 7 mol% and 9 mol%, at a total molar flow rate (amount of all gas particles in mol) of, for example, 24 000 to 28 000 kmol/h of reactant gas, corresponding to about 100 000 kg/h to 200 000 kg/h of ethylene. The cycle gas apparatus 1 is configured such that, in addition, a ballast gas, for example methane, is used, which can be introduced into the cycle gas in one or more gas inlets 15, 16.

Argon accumulates in the cycle gas during the operation of the cycle gas apparatus and has to be removed. The cycle gas apparatus 1 therefore optionally has a gas purge for argon, which limits the concentration to from 6% by volume to 10% by volume; this is typically a valve for gas discharge, which may be configured with or without a membrane for recovery of ethylene.

The reactant gas also possibly contains small proportions of water and ethanol, for example less than 5000 ppm of water and less than 500 ppm of ethanol. The presence of ethanol in the reactant gas 17 is undesirable since it deactivates the catalyst, and it is an object of the invention to minimize the ethanol content as far as possible.

The first separator stage 7 is typically an ethylene oxide absorber and serves to separate off the ethylene oxide product in order to provide a product stream 10 with which the ethylene oxide product is discharged from the cycle gas apparatus 1. The first separator stage 7 additionally also serves to separate off by-products, especially including water and aldehydes. The cycle gas may be cooled down to about 50 to 60°C prior to entry into the first separator stage, for example by means of a heat exchanger. The first separator stage 7 has at least one inlet, an outlet and an exit; the inlet serves to admit the ethylene oxide-containing cycle gas 6 from the reactor into the separator stage 7, the outlet serves for recycling of the cycle gas 8 from the separator stage 7 that remains after ethylene oxide has been separated off into the cycle gas apparatus 1, and the exit is an apparatus for discharge of the product stream 10. More preferably, the first separator stage 7 additionally has an inlet by which a scrubbing medium, for example water, can be introduced into the separator stage. The inlet to the first separator stage 7 is in direct fluid contact with the outlet from the epoxidation reactor 2, such that the product gas 3, i.e. the ethylene oxide-containing cycle gas, can be guided into the first separator stage 7. The outlet can be used to return the remaining gases, i.e. the cycle gas from which the ethylene oxide has been removed at least in part, typically down to a content of below 50 ppm (based on volume), to the cycle gas stream.

The first separator stage 7 is typically configured as a gas scrubber, for example as an absorption column operated in countercurrent, which is configured such that the ethylene oxide-containing cycle gas 6 can be introduced at the inlet at the base of the absorption column, can flow through said absorption column and can reach the outlet from the absorption column at the top of the absorption column. The absorption column permits introduction of scrubbing water 9 at the top of the absorption column at about 20°C at about 1.5 to 2 times the mass flow rate relative to the cycle gas during operation. The absorption column is also configured such that this scrubbing water 9 can be introduced at the top of the absorption column and flows within the column to the base of the column. The absorption column has internals (packings, trays, etc.) that increase the exchange area as the scrubbing water 9 flows through the absorption column. During operation, the scrubbing water 9 comes into contact with the cycle gas, absorbs ethylene oxide, which has good solubility, and forms the product stream 10. In flow direction of the product stream 10, this is followed by an apparatus which permits the separating-off of ethylene oxide, for example a stripper, and which permits recycling of the cleaned water to the absorber, while the ethylene oxide can be processed further downstream, in order to remove the typical impurities (especially water, ethanol, glycols).

The ethylene oxide that forms the product stream 10 is in a crude form, meaning that it may be diluted in water and may contain further impurities. More particularly, the ethylene oxide from the product stream 10 may be contaminated with the permanent gases in the cycle gas process, since, during the operation, for example, of an absorption column as the first separator stage 7, smaller amounts of the gases in the cycle gas process (CO₂, argon, ethylene, ethane, methane and O₂) are typically also removed from the process. More preferably, the cycle gas apparatus 1 therefore includes an apparatus that permits "reclaiming", i.e. the separating of the ethylene, methane and O₂ gases from the product stream 10 and the return of these reclaimed gases to the cycle gas, for example downstream of the separator stage 7 by means of a reclaim compressor. For this purpose, for example, it is possible to use a reabsorber which permits scrubbing of the product stream 10 with water downstream of the stripper, absorbing of the ethylene oxide and separating-off of the reclaim gases.

The first separator stage 7 is optionally followed by a gas purge for argon. This may be followed in cycle gas stream direction by a cycle gas compressor 11 that enables compensation for the pressure drop during the operation of the cycle gas apparatus. A cycle gas compressor 11 may alternatively also be used anywhere else in the cycle gas apparatus 1, provided that it is capable of increasing the pressure of the cycle gas.

The second separator stage 12 is typically a CO₂ absorber and serves to separate the CO₂ 13 from the cycle gas. The cycle gas may be preheated here to the operating temperature of the absorber of 60°C to 80°C, for example with the aid of a heat exchanger. The second separator stage 12 has at least one inlet, an outlet and an exit; the CO₂-containing cycle gas is introduced into the inlet, the CO₂ 13 that has been separated off is discharged from the cycle gas apparatus 1 by the exit, and the cycle gas 14 that has been freed of CO₂ is introduced back into the cycle gas apparatus 1 by the outlet. In addition, there may be an inlet for a scrubbing medium and an outlet for the CO2-enriched scrubbing medium. The cycle gas apparatus 1 is configured such that the cycle gas 8 that has been freed of ethylene oxide and leaves the first separator stage 7 via the outlet can be guided to the inlet of the second separator stage 12; in other words, outlet from the first separator stage 7 and inlet to the second separator stage 12 are in direct fluid contact.

The second separator stage 12 is typically configured as a gas scrubber, for example as an absorption column operated in countercurrent, which is configured such that the CO₂-containing cycle gas can be introduced at the inlet at the base of the absorption column, can flow through said absorption column and can reach the outlet from the absorption column at the top of the absorption column. During operation, at the same time, it is possible to introduce scrubbing water at the top of the absorption column at about 50°C to 70°C and at about 1.5 to 2 times the mass flow rate relative to the cycle gas. The absorption column is configured such that this scrubbing water can be introduced at the top of the absorption column and flows within the column to the base of the column. The absorption column has internals which increase the exchange area (packings, trays, etc.) as the scrubbing water (typically containing an active species such as K₂CO₃ in order to increase the CO₂ absorption capacity) flows through the absorption column. During operation, the scrubbing water comes into contact with the cycle gas, and absorbs CO₂, which has good solubility.

The cycle gas apparatus 1 of the invention is configured such that the outlet from the second separator stage 12 is in direct fluid contact with the inlet to the epoxidation reactor 2, such that the cycle gas that has been freed of ethylene oxide and CO₂ can be guided into the epoxidation reactor 2. Between the outlet from the second separator stage and the inlet to the epoxidation reactor 2, the cycle gas apparatus 1 typically has one or more gas inlets 15, 16 that permit introduction of oxygen and a ballast gas, typically methane, together or as a gas mixture, into the cycle gas. However, the one or more gas inlets 15, 16 may be mounted at any suitable point in the cycle gas process.

It is essential to the invention that there is a reactant stream inlet 4 between the outlet from the epoxidation reactor 2 and the first separator stage 7, which permits introduction of ethylene into the cycle gas apparatus 1, meaning that the cycle gas apparatus 1 is configured such that the ethylene reactant (in flow direction of the cycle gas) is introduced downstream of the epoxidation reactor 2 and upstream of the first separator stage 7. This can also be effected with the aid of a compressor in order to adjust the ethylene to the pressure in the cycle gas and in order thus to enable introduction.

The invention also relates to the operation of the cycle gas apparatus 1 of the invention in the context of the cycle gas process of the invention, i.e. a process for preparing ethylene oxide from ethylene with the cycle gas apparatus 1 according to the invention, wherein a reactant stream 5 is introduced into the cycle gas apparatus 1, and a product stream 10 is obtained.

More particularly, the process for preparing ethylene oxide may be a cycle gas process which is performed in the cycle gas apparatus 1 of the invention, comprising the steps of:
a) converting a reactant gas 17 to ethylene oxide in the epoxidation reactor 2,
b) introducing the ethylene-containing reactant stream 5 at the reactant stream inlet 4 into the product gas 3 in order to obtain an ethylene-containing cycle gas,
c) separating the ethylene oxide from the ethylene-containing cycle gas from step b) with the first separator stage 7 in order to provide an ethylene oxide-containing product stream 10,
d) separating CO₂ from the cycle gas 8 from step c) with the second separator stage 12,
e) introducing O₂ and a ballast gas into the cycle gas at one or more gas inlets 15, 16,
f) returning the resultant cycle gas as reactant gas 17 to step a).

Steps a) to f) here are typically performed simultaneously and continuously. In the operation of the cycle gas process of the invention, different gas compositions are formed at different points.

The reactant gas 17 may include between 21 mol% and 51 mol%, more preferably between 31 mol% and 41 mol% and even more preferably 35 mol% to 37 mol% of ethylene, and 3 mol% to 13 mol%, preferably 3 mol% to 12 mol%, more preferably between 5 mol% and 11 mol% and especially preferably between 7 mol% and 9 mol% of O₂. In the case of a plant having a capacity of about 200 kt/a of ethylene oxide, this results in a total molar flow rate (amount of all gas particles in mol) of, for example, 24 000 kmol/h to 28 000 kmol/h, more preferably 26 000 kmol/h to 27 000 kmol/h. In addition, the reactant gas typically contains between 6 mol% and 10 mol% of Ar, between 3 mol% and 7 mol% of N₂, and less than 0.5 mol% each of CO₂ and H₂O. In the cycle gas process of the invention, the ethanol content is typically below one ppm. The reactant gas in the cycle gas process typically has a temperature in the range from 100°C to 200°C, more preferably from 120°C to 160°C, and has the pressure required for the epoxidation reactor 2, i.e., for example, 11 bar to 31 bar, more preferably 19 bar to 23 bar.

The epoxidation reactor 2 is operated at a pressure between ambient pressure and up to 50 bar (absolute pressure), preferably at an absolute pressure between 11 bar and 31 bar. The temperature in the reactor during the reaction here is 150°C to 350°C, preferably 200°C to 300°C. The epoxidation reactor 2 typically shows an activity of 5% to 10% and a selectivity of 80% to 90%, such that a product gas 3 is obtained.

The composition of the product gas 3, i.e. of the cycle gas downstream of the epoxidation reactor 2 that contains the crude ethylene oxide, is dependent on the conversion in the reactor (activity, selectivity) and the composition of the reactant stream 5, and contains, for example, 19 mol% to 49 mol%, more preferably 29 mol% to 39 mol% and even more preferably 32 mol% to 35 mol% of ethylene, and 1 mol% to 10 mol%, preferably 2 mol% to 8 mol%, especially preferably 4 mol% to 6 mol% of O₂. The total molar flow rate, depending on the conversion in the epoxidation reactor 2, is reduced by a magnitude of between 100 and 500 kmol/h. Otherwise, the reactant gas contains typically between 6 mol% and 10 mol% of Ar and between 3 and 7 mol% of N₂, and the reaction by-products CO₂ and H₂O in a content of 0.5 mol% to 1.5 mol% of CO₂ and 0.5 mol% to 1.5 mol% of H₂O. The pressure of the cycle gas is reduced by the pressure drop in the reactor to a magnitude of, for example, 10 bar to 30 bar, typically 18 bar to 22 bar.

The reactant stream 5, i.e. the ethylene-containing gas that is introduced at the reactant stream inlet 4 between reactor outlet and second separator stage, for operation of the process of the invention, may consist of 100% ethylene. But the advantages of the invention are also realized when the ethylene is contaminated, for example with ethanol that remains as an impurity in the ethylene in the dehydration of biobased ethanol to ethylene, together with water. The invention is therefore particularly suitable for conversion of ethylene that has been obtained by the dehydration of ethanol.

After the dehydration of ethanol, the ethanol can be purified by a flash, which is preferably operated at standard pressure to absolute pressure 5 bar and a temperature between 15 and 40°C.

The reactant stream 5 preferably contains ethylene only to an extent of more than 90 mol%, more preferably more than 95 mol%, most preferably more than 98 mol%. The reactant stream 5 therefore preferably contains water and ethanol, especially up to 5 mol%, more preferably up to 3 mol% and especially preferably 0.5 mol% of ethanol, and otherwise essentially water as an impurity. The molar ethanol/water ratio in the reactant stream 5 is more preferably between 0.01 and 1, especially preferably between 0.02 and 0.5. The molar flow rate of the reactant stream is between 500 kmol/h and 1000 kmol/h, more preferably 500 kmol/h to 700 kmol/h, with a pressure corresponding approximately to the pressure in the cycle gas process at this point.

Typically, the cycle gas, after the ethylene has been fed in, consists of a gas mixture 6 containing 21 mol% to 51 mol%, more preferably 31 mol% to 41 mol% and especially preferably 34 mol% to 38 mol% of ethylene, and 1 mol% to 10 mol%, preferably 2 mol% to 8 mol% and more preferably between 4 mol% and 6 mol% of O₂, below 0.3 mol% of H₂O, 6 mol% to 10 mol% of Ar, 3 mol% to 8 mol% of N₂, below 1 mol% of CO₂ and 1 mol% to 3 mol% of ethylene oxide, the balance being methane. The pressure in the cycle gas process at this point corresponds approximately to the pressure upstream of the inlet for the recycle stream 5. The total molar flow rate increases through the introduction of the ethylene by the corresponding magnitude, for example by 200 to 600 kmol/h, preferably 300 kmol/h to 500 kmol/h.

Typically, the cycle gas 8, between the separator stages 7 and 12, or if applicable between the reclaim gas inlet and the second separator stage 12, consists of a gas mixture containing 22 mol% to 52 mol% and more preferably 32 mol% to 41 mol% of ethylene, especially preferably 35 mol% to 38 mol%, and 1 to 10 mol%, preferably 2 mol% to 8 mol% and more preferably between 4 mol% and 6 mol% of O₂, below 0.3 mol% of H₂O, 6 mol% to 10 mol% of Ar, 3 mol% to 7 mol% of N₂ and below 1 mol% of CO₂, the balance being methane.

In the cycle gas process, the inlet for the reactant stream 5 follows downstream, in the gas direction, of the first separator stage 7 that removes the crude ethylene oxide from the cycle gas and provides it in the reactant stream 5.

The ethylene oxide is separated off to form the product stream 10 in the first separator stage 7. The latter is preferably configured as an absorption column and is preferably operated at an elevated pressure between 15 bar and 25 bar (absolute) and at a temperature between 10°C and 60°C. The first separator stage 7 is typically configured in two-part form, with absorption of the ethylene oxide by the scrubbing water in an upper region and of aldehydes and water in a lower region.

If a reclaim process is being implemented, the product stream 10 containing the ethylene oxide is scrubbed to free it of the remaining gases, for example with water in a reabsorber, and the permanent gases (ethylene, methane and oxygen) can optionally be recycled into the process via a reclaim compressor. This reclaim stream consists mainly of methane and ethylene, with an expected mass flow rate in an industrial context of, for example, about 500 kg/h in a 200 kt/a ethylene oxide plant. This shows the order of magnitude of the reclaim stream, which does not have any noticeable effect on the concentrations in the cycle gas, since amounts of these two species (methane and ethylene) of about 150 t/h each are encountered here.

The cycle gas 8 that remains after ethylene oxide has been separated off is supplied to the second separator stage 12, with removal of the CO₂ by-product. The CO₂ absorber likewise works in countercurrent in a column with water. Another active species (e.g. K₂CO₃) is additionally used here in order to remove more CO₂ from the gas. This column is also operated at higher temperature in order to accelerate the kinetics for the reaction of active species with CO₂ (about 50 to 70°C). However, this higher temperature leads to poorer solubility in relation to other gases, for example ethanol. Connected beyond the absorber is a desorber, including in the case of the CO₂ absorber. This regenerates the active species and removes the CO₂ from the process, for example by thermal breakdown of K₂CO₃.

Between the second separator stage 12 and epoxidation reactor 2, argon is typically separated off by a gas purge, and oxygen and the methane ballast gas are introduced in order to compensate for the loss of the two gases in the cycle gas process (through oxidation in the reactor and via the separator stage). The introduction can be effected by one inlet, or separately by two or more inlets, for example one each for the methane ballast gas 15 and oxygen 16. However, it is also possible to separate the argon off and to admit the methane ballast gas and oxygen at any other suitable point in the cycle gas process.

The advantage of the cycle gas process of the invention and hence of the proposed cycle gas apparatus 1 is that the cycle gas process is more tolerant with regard to impurities in the ethylene reactant stream 5. This is especially important in the case of development of ethylene from biological sources. For example, the ethylene may be obtained by the dehydration of ethanol that has been obtained by a biological method in a preceding step. The dehydration of ethanol typically gives rise to by-products that can completely deactivate the silver catalyst used in the epoxidation step even in small concentrations, especially including unconverted ethanol and some hydrocarbons (e.g. C₂-C₆ alkanes and alkenes). It has been recognized in the context of the invention that the ethylene oxide absorber has separation performance with regard to these by-products. By virtue of the introduction according to the invention, i.e. the feeding-in of the ethylene reactant downstream of the epoxidation reactor 2, it is possible to utilize this separation performance in order to purify the cycle gas which is guided into the epoxidation reactor 2. The effect of this is that the purification of the ethylene obtained from biological sources is no longer necessary, or can have a less complex configuration.

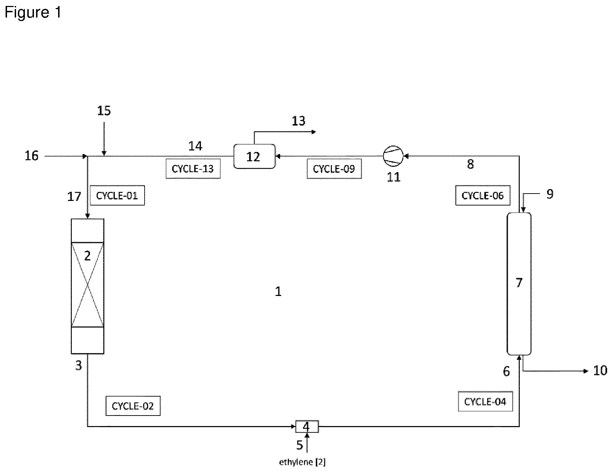

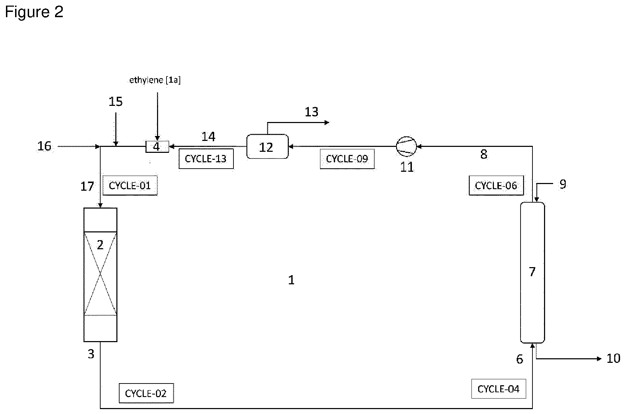

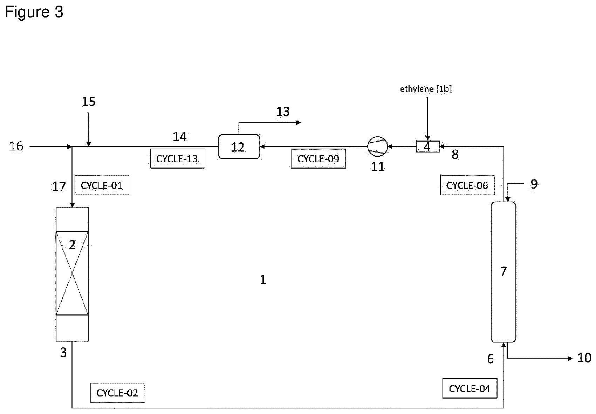

Positioning of the reactant stream inlet 4 upstream of the epoxidation reactor 2 (see figure 2, inlet [1a]) apparently leads to loading of the reactant gas, for example with ethanol from the reactant stream. Positioning of the reactant stream inlet 4 between the separate stages, i.e. upstream of the second separator stage in flow direction (see figure 3, inlet [1b]), by contrast, does not lead to the separation of ethanol, for example, together with the CO₂, as apparent from the simulations in the examples. If, by contrast, the reactant stream inlet 4, for introduction of the ethylene, is positioned in accordance with the invention between the epoxidation reactor 2 (see figure 1, inlet [2]) and the first separator stage, ethanol, for example, is discharged together with the ethylene oxide.
Figure 1: Inventive cycle gas system having reactant stream inlet 4 at position [2].
Figure 2: Noninventive cycle gas system having reactant stream inlet 4 at position [1a].
Figure 3: Noninventive cycle gas system having reactant stream inlet 4 at position [1b].

### Examples

A cycle gas process 1 as shown in figure 1 was simulated with the Aspen+ simulation software (v 10.2), and the concentration was examined at selected sites. The simulation was calculated with UNIFAC parameters with Henry coefficients. Water was simulated by means of steam tables. The simulation proceeded from an overall capacity of 200 kt/year of ethylene oxide equivalents (EOE) and a load of 8000 h/a, corresponding to 25 000 kg/h of EOE. The reactant stream 5 ("feed") here consisted exclusively of ethylene, water and ethanol. Operating parameters assumed for the ethylene oxide reactor 2 for oxidation of the ethylene to ethylene oxide were a pressure of 20 bar and a temperature of 240°C. The cycle gas is cooled down to 56°C upstream of the first separator stage. The first separation stage in the simulation consists of two subunits. The first consists of four theoretical plates and serves for the removal of aldehyde and water. The second substage is the actual EO absorber, having 10 theoretical plates. The Ar bleed removes a constant 500 kg/h of cycle gas. The Ar bleed is followed by the recycling of the reclaim, the cycle gas compressor and a heat exchanger (70°C) for the second separator stage 12 (CO₂ absorber), having a pressure of 21 bar and at 70°C. This stage is simulated as an absorber having eight theoretical plates and a CO₂ removal of 95%. Downstream of the second separator stage, CH₄ and O₂ are fed into the cycle gas. The amount of O₂ is adjusted so as to result in 7.5% by volume of O₂ at the reactor inlet; the amount of CH₄ regulates the ethylene concentration to 36% by volume at the reactor inlet. Before entering the reactor, the cycle gas is preheated to 140°C by means of a heat exchanger.

In order to assume realistic concentrations of ethanol and water in the reactant stream 5, the dehydration of ethanol followed by purification by a flash stage and a compressor was simulated. The catalytic EtOH dehydration was simulated here at 2 bar and 420°C, and the flash before the feeding of ethylene was operated at a simulated 2 bar and a temperature of 20°C. The reactant stream 5 thus obtained was used for the simulation of the cycle gas process 1.

As well as the cycle gas compressor 11, a compressor between flash and cycle gas was simulated in order to adjust the pressure. Neither compressor has any effect on the gas composition; they merely increase the pressure and temperature. The simulation additionally took account of the first separator stage 7, the reclaim gas stream and the second separator stage 12. The first separator stage 7 for separating off the ethylene oxide was simulated as an absorption column, such that crude ethylene oxide was obtained in a product stream 10 in the simulation.

The simulation of the cycle gas takes account of ethylene (C₂H₄), methane (CH₄) as circulation ballast, oxygen (O₂), water (H₂O), ethanol (EtOH), ethylene oxide (EO), nitrogen (N₂) and argon (Ar). The simulated inventive feeding of the reactant stream 5 at position [2] was compared with the simulated noninventive feeding of the reactant stream at positions [1a] and [1b].

Proceeding from a 100 percent ethanol stream, in examples 1 to 3, a conversion in the dehydration reactor to ethylene of 97% (example 1), 99% (example 2) and 99.5% (example 3) was assumed. Table 1 shows the proportion of water and ethanol upstream of the epoxidation reactor (CYCLE-01), in these three scenarios and in the case of an inventive input of the reactant stream 1 at position [2] compared to the two noninventive inputs at positions [1a] and [1b].

**Table 1: Simulation results: Concentrations of water and ethanol upstream of the epoxidation reactor (CYCLE-01).**

| Scenario | Position | H₂O mol% | EtOH ppm |
|---|---|---|---|
| 97% ethanol | | | |
| conversion | [1a] | 0.27 | 122 |
| Comparative example 1 | [1b] | 0.25 | 33 |
| Comparative example 2 | [2] | 0.25 | < 1 |
| Example 1 | | | |
| | | | |
| 99% ethanol | | | |
| conversion | [1a] | 0.27 | 47 |
| Comparative example 3 | [1b] | 0.25 | 13 |
| Comparative example 4 | [2] | 0.25 | < 1 |
| Example 2 | | | |
| | | | |
| 99.5% ethanol | | | |
| conversion | [1a] | 0.27 | 24 |
| Comparative example 5 | [1b] | 0.25 | 7 |
| Comparative example 6 | [2] | 0.25 | < 1 |
| Example 3 | | | |

It can be inferred from the results in table 1 that introduction of the ethylene at position 2 is preferable over introduction at position [1a] or [1b], since the EtOH load on the epoxidation catalyst is distinctly reduced. In addition, introduction at position [2], even in the case of poorer conversion of the ethanol to ethylene, can ensure loading of the epoxidation catalyst with ethanol of < 1 ppm.

The critical composition directly upstream of the epoxidation reactor (CYCLE-01) is shown in table 1 for all scenarios; table 2 shows the composition of the various streams of matter for the simulation according to example 2.

**Table 2: Molar flow rate, temperature, pressure and substance concentrations in the cycle gas at a simulated ethanol conversion of 99% in the dehydration.**

| Stream | Scen ario | Flow rate kmol/h | Temperatur e °C | Press ure bar | Concentrations mol% | | | | | | | | |
|---|---|---|---|---|---|---|---|---|---|---|---|---|---|
| | | | | | *Ethylene (C₂H₄)* | *Oxygen (O₂)* | *Methane (CH₄)* | *Ethylene oxide (EO)* | *Ethanol (EtOH)* | *Argon (Ar)* | *Nitrogen (N₂)* | *Carbon dioxide (CO₂)* | *Water (H₂O)* |
| CYCLE-01 | [1a] | 26263.8 | 140.0 | 21 | 36.00 | 7.50 | 44.78 | 0.00 | 47 ppm | 6.77 | 4.59 | 0.08 | 0.27 |
| | [1b] | 26262.8 | 140.0 | 21 | 36.00 | 7.50 | 44.27 | 0.00 | 13 ppm | 7.06 | 4.85 | 0.08 | 0.25 |
| | [2] | 26252.5 | 140.0 | 21 | 36.00 | 7.50 | 43.27 | 0.00 | <1 ppm | 7.77 | 5.14 | 0.08 | 0.25 |
| CYCLE-02 | [1a] | 25980.1 | 240.0 | 20 | 33.84 | 5.40 | 45.27 | 2.18 | 47 ppm | 6.85 | 4.64 | 0.81 | 1.00 |
| | [1b] | 25979.2 | 240.0 | 20 | 33.84 | 5.40 | 44.75 | 2.18 | 13 ppm | 7.13 | 4.91 | 0.81 | 0.98 |
| | [2] | 25969.0 | 240.0 | 20 | 33.84 | 5.40 | 43.74 | 2.18 | <1 ppm | 7.86 | 5.19 | 0.81 | 0.98 |
| CYCLE-03: | [1a] | 25980.1 | 240.0 | 20 | 33.84 | 5.40 | 45.27 | 2.18 | 47 ppm | 6.85 | 4.64 | 0.81 | 1.00 |
| | [1b] | 25979.2 | 240.0 | 20 | 33.84 | 5.40 | 44.75 | 2.18 | 13 ppm | 7.13 | 4.91 | 0.81 | 0.98 |
| | [2] | 26649.1 | 240.0 | 20 | 35.50 | 5.26 | 42.62 | 2.13 | 46 ppm | 7.66 | 5.06 | 0.79 | 0.98 |
| CYCLE-04 | [1a] | 25980.1 | 55.0 | 20 | 33.84 | 5.40 | 45.27 | 2.18 | 47 ppm | 6.85 | 4.64 | 0.81 | 1.00 |
| | [1b] | 25979.2 | 55.0 | 20 | 33.84 | 5.40 | 44.75 | 2.18 | 13 ppm | 7.13 | 4.91 | 0.81 | 0.98 |
| | [2] | 26649.1 | 55.0 | 20 | 35.50 | 5.26 | 42.62 | 2.13 | 46 ppm | 7.66 | 5.06 | 0.79 | 0.98 |
| CYCLE-05 | [1a] | 25931.2 | 49.9 | 17 | 33.91 | 5.41 | 45.36 | 2.18 | 46 ppm | 6.86 | 4.65 | 0.81 | 0.82 |
| | [1b] | 25936.8 | 49.9 | 17 | 33.90 | 5.41 | 44.82 | 2.18 | 13 ppm | 7.15 | 4.91 | 0.81 | 0.82 |
| | [2] | 26605.2 | 49.9 | 17 | 35.55 | 5.27 | 42.69 | 2.12 | 45 ppm | 7.67 | 5.07 | 0.79 | 0.83 |
| CYCLE-06 | [1a] | 25168.5 | 20.2 | 16 | 34.87 | 5.57 | 46.71 | 0.00 | <1 ppm | 7.07 | 4.79 | 0.82 | 0.18 |
| | [1b] | 25174.8 | 20.2 | 16 | 34.86 | 5.57 | 46.15 | 0.00 | <1 ppm | 7.36 | 5.06 | 0.82 | 0.18 |
| | [2] | 25837.0 | 20.2 | 16 | 36.54 | 5.42 | 43.94 | 0.00 | <1 ppm | 7.90 | 5.22 | 0.80 | 0.18 |
| CYCLE-07 | [1a] | 25147.4 | 20.2 | 16 | 34.87 | 5.57 | 46.71 | 0.00 | <1 ppm | 7.07 | 4.79 | 0.82 | 0.18 |
| | [1b] | 25154.2 | 20.2 | 16 | 34.86 | 5.57 | 46.16 | 0.00 | <1 ppm | 7.36 | 5.06 | 0.82 | 0.18 |
| | [2] | 25816.2 | 20.2 | 16 | 36.54 | 5.42 | 43.94 | 0.00 | <1 ppm | 7.90 | 5.22 | 0.80 | 0.18 |
| CYCLE-08 | [1a] | 25194.4 | 20.6 | 16 | 34.91 | 5.56 | 46.67 | 0.00 | <1 ppm | 7.06 | 4.78 | 0.84 | 0.18 |
| | [1b] | 25881.8 | 26.7 | 16 | 36.58 | 5.42 | 44.90 | 0.00 | 47 ppm | 7.16 | 4.92 | 0.81 | 0.21 |
| | [2] | 25864.0 | 20.6 | 16 | 36.59 | 5.42 | 43.90 | 0.00 | <1 ppm | 7.89 | 5.21 | 0.81 | 0.18 |
| CYCLE-09 | [1a] | 25194.4 | 50.9 | 22 | 34.91 | 5.56 | 46.67 | 0.00 | <1 ppm | 7.06 | 4.78 | 0.84 | 0.18 |
| | [1b] | 25881.8 | 57.2 | 22 | 36.58 | 5.42 | 44.90 | 0.00 | 47 ppm | 7.16 | 4.92 | 0.81 | 0.21 |
| | [2] | 25864.0 | 50.9 | 22 | 36.59 | 5.42 | 43.90 | 0.00 | <1 ppm | 7.89 | 5.21 | 0.81 | 0.18 |
| CYCLE-10 | [1a] | 25194.4 | 70.0 | 22 | 34.91 | 5.56 | 46.67 | 0.00 | <1 ppm | 7.06 | 4.78 | 0.84 | 0.18 |
| | [1b] | 25881.8 | 70.0 | 22 | 36.58 | 5.42 | 44.90 | 0.00 | 47 ppm | 7.16 | 4.92 | 0.81 | 0.21 |
| | [2] | 25864.0 | 70.0 | 22 | 36.59 | 5.42 | 43.90 | 0.00 | <1 ppm | 7.89 | 5.21 | 0.81 | 0.18 |
| CYCLE-11 | [1a] | 25571.8 | 70.0 | 21 | 34.35 | 5.48 | 45.96 | 0.00 | <1 ppm | 6.95 | 4.71 | 0.82 | 1.74 |
| | [1b] | 26262.1 | 70.0 | 21 | 36.00 | 5.34 | 44.23 | 0.00 | 16 ppm | 7.05 | 4.85 | 0.80 | 1.74 |
| | [2] | 26251.6 | 70.0 | 21 | 36.00 | 5.34 | 43.23 | 0.00 | <1 ppm | 7.77 | 5.13 | 0.80 | 1.74 |
| CYCLE-12 | [1a] | 25383.5 | 70.0 | 21 | 34.60 | 5.52 | 46.30 | 0.00 | <1 ppm | 7.00 | 4.75 | 0.08 | 1.75 |
| | [1b] | 26073.9 | 70.0 | 21 | 36.26 | 5.37 | 44.55 | 0.00 | 17 ppm | 7.10 | 4.88 | 0.08 | 1.75 |
| | [2] | 26063.5 | 70.0 | 21 | 36.26 | 5.37 | 43.54 | 0.00 | <1 ppm | 7.82 | 5.17 | 0.08 | 1.75 |
| CYCLE-13 | [1a] | 25002.2 | 30.0 | 21 | 35.13 | 5.60 | 47.00 | 0.00 | <1 ppm | 7.11 | 4.82 | 0.08 | 0.25 |
| | [1b] | 25682.1 | 30.0 | 21 | 36.81 | 5.46 | 45.23 | 0.00 | 13 ppm | 7.21 | 4.96 | 0.08 | 0.25 |
| | [2] | 25672.1 | 30.0 | 21 | 36.81 | 5.46 | 44.21 | 0.00 | <1 ppm | 7.94 | 5.25 | 0.08 | 0.25 |
| CYCLE-14 | [1a] | 25682.4 | 36.6 | 21 | 36.81 | 5.46 | 45.76 | 0.00 | 48 ppm | 6.92 | 4.69 | 0.08 | 0.28 |
| | [1b] | 25682.1 | 30.0 | 21 | 36.81 | 5.46 | 45.23 | 0.00 | 13 ppm | 7.21 | 4.96 | 0.08 | 0.25 |
| | [2] | 25672.1 | 30.0 | 21 | 36.81 | 5.46 | 44.21 | 0.00 | <1 ppm | 7.94 | 5.25 | 0.08 | 0.25 |
| CYCLE-15 | [1a] | 25692.5 | 36.6 | 21 | 36.80 | 5.45 | 45.78 | 0.00 | 48 ppm | 6.92 | 4.69 | 0.08 | 0.28 |
| | [1b] | 25691.6 | 30.0 | 21 | 36.80 | 5.45 | 45.25 | 0.00 | 13 ppm | 7.21 | 4.96 | 0.08 | 0.25 |
| | [2] | 25681 .3 | 30.0 | 21 | 36.80 | 5.45 | 44.23 | 0.00 | <1 ppm | 7.94 | 5.25 | 0.08 | 0.25 |
| CYCLE-16 | [1a] | 26263.8 | 36.3 | 21 | 36.00 | 7.50 | 44.78 | 0.00 | 47 ppm | 6.77 | 4.59 | 0.08 | 0.27 |
| | [1b] | 26262.8 | 29.8 | 21 | 36.00 | 7.50 | 44.27 | 0.00 | 13 ppm | 7.06 | 4.85 | 0.08 | 0.25 |
| | [2] | 26252.3 | 29.8 | 21 | 36.00 | 7.50 | 43.27 | 0.00 | <1 ppm | 7.77 | 5.14 | 0.08 | 0.25 |

**Table 3: Process stream descriptions**

| Process stream | Description |
|---|---|
| CYCLE-01 | Input to EO reactor |
| CYCLE-02 | Output from EO reactor |
| CYCLE-03 | Downstream of the reactant stream inlet [2]* in flow direction |
| CYCLE-04 | Downstream of heat exchanger and upstream of EO absorber (56°C) in flow direction |
| CYCLE-05 | Between lower and upper stages of the EO absorber |
| CYCLE-06 | Upper gas output from EO absorber |
| CYCLE-07 | Downstream of Ar bleed |
| CYCLE-08 | Beyond reclaim and reactant stream inlet [1b]* in flow direction |
| CYCLE-09 | Downstream of cycle gas compressor in flow direction |
| CYCLE-10 | Downstream of heat exchanger (70°C) and upstream of CO₂ absorber in flow direction |
| CYCLE-11 | Downstream of CO₂ absorber (column) in flow direction |
| CYCLE-12 | Downstream of CO₂ absorber (CO₂ removal) in flow direction |
| CYCLE-13 | Downstream of CO₂ absorber flash (30°C, 21 bar) in flow direction |
| CYCLE-14 | Downstream of the reactant stream inlet [1a]* in flow direction |
| CYCLE-15 | Downstream of addition of ballast gas (CH₄) in flow direction |
| CYCLE-16 | Downstream of addition of O₂ and upstream of CYCLE-01 in flow direction |

| | |
|---|---|
| *if applicable | |

## Claims

1. Cycle gas apparatus 1 for preparation of ethylene oxide from ethylene in a cycle gas process, comprising:
a) an epoxidation reactor 2 containing a catalyst suitable for conversion of ethylene to ethylene oxide, having an inlet for a reactant gas and an outlet for the product gas 3,
b) a first separator stage 7 suitable for removing ethylene oxide from the cycle gas process and providing it in a product stream 10, where the first separator stage 7 has an inlet connected to the outlet from the epoxidation reactor 2 and has an outlet by which the remaining gases can be returned to the cycle gas process,
c) a second separator stage 12 suitable for removing CO₂ from the cycle gas process, where the second separator stage 12 has an inlet which is connected to the outlet of the first separator stage 7 and has an outlet by which the remaining gases can be returned to the cycle gas process,
d) one or more gas inlets 15, 16 that allow introduction of O₂ and a ballast gas into the cycle gas apparatus 1,
**characterized in that** there is a reactant stream inlet 4 between the outlet from the epoxidation reactor 2 and the first separator stage 7, which permits the introduction of ethylene into the cycle gas apparatus 1.

2. Cycle gas apparatus 1 according to Claim 1, **characterized in that** the catalyst in the epoxidation reactor 2 is an Al₂O₃-supported Ag catalyst.

3. Cycle gas apparatus 1 according to either of Claims 1 and 2, **characterized in that** the first separator stage 7 is an ethylene oxide absorber configured as an absorption column operated in countercurrent.

4. Cycle gas apparatus 1 according to any of the preceding claims, **characterized in that** the outlet from the second separator stage 12 is connected to the inlet for the reactant gas from the epoxidation reactor 2, in order to enable recycling of the cycle gas as reactant gas.

5. Cycle gas apparatus 1 according to any of the preceding claims, **characterized in that** the cycle gas apparatus 1 has a reclaim device which allows ethylene and methane from the product stream 10 to be introduced back into the cycle gas process.

6. Cycle gas apparatus 1 according to any of the preceding claims, **characterized in that** the second separator stage 12 is a CO₂ absorber configured as an absorption column operated in countercurrent.

7. Cycle gas apparatus 1 according to any of the preceding claims, **characterized in that** one gas inlet 15 or the plurality of gas inlets 15, 16 that allow introduction of oxygen and a ballast gas into the cycle gas apparatus 1 is/are between the outlet from the second separator stage 12 and the inlet to the epoxidation reactor 2.

8. Cycle gas process for preparing ethylene oxide from ethylene, in which a cycle gas apparatus 1 is operated according to any of Claims 1 to 7, by introducing an ethylene-containing reactant gas stream at the reactant stream inlet 4.

9. Cycle gas process according to Claim 8, comprising the steps of:
a) converting a reactant gas 17 to ethylene oxide in the epoxidation reactor 2,
b) introducing the reactant stream 5 comprising ethylene at the reactant stream inlet 4 into the product gas 3 in order to obtain an ethylene-containing cycle gas,
c) separating the ethylene oxide from the ethylene-containing cycle gas from step b) with the first separator stage 7 in order to provide an ethylene oxide-containing product stream 10,
d) separating CO₂ from the cycle gas 8 from step c) with the second separator stage 12,
e) introducing O₂ and a ballast gas into the cycle gas at one or more gas inlets 15, 16, and
f) returning the resultant cycle gas as reactant gas 17 to step a).

10. Cycle gas process according to Claim 9, **characterized in that** the ethylene fed in is contaminated with up to 5 mol% of ethanol.
